# EUROPÄISCHE PATENTANMELDUNG

(11) **EP 3 881 862 A1**
(43) Veröffentlichungstag der Anmeldung: **22.09.2021**
(21) Anmeldenummer: 20163836.8
(22) Anmeldetag: 18.03.2020
(51) Int. Cl.: A61K 38/46, A61P 13/12, C12N 9/22, G01N 33/68

(54) **POLYPEPTID ZUR THERAPIE VON GLOMERULÄREN NIERENERKRANKUNGEN UND ANALYSE DES VERLAUFES UND PROGNOSE DER ABHÄNGIGEN SYNDROME**

(71) Anmelder: Pharis Biotec GmbH, 30625 Hannover (DE); Friedrich-Alexander-Universität Erlangen-Nürnberg, 91054 Erlangen (DE)
(72) Erfinder: Forssmann, Wolf-Georg, 30559 Hannover (DE); Mario, Schiffer, 91054 Erlangen (DE)
(74) Vertreter: dompatent von Kreisler Selting Werner - Partnerschaft von Patent- und Rechtsanwälten mbB

(57) **Zusammenfassung**

Es wird ein Arzneimittel enthaltend humane Ribonuclease 1 offenbart. Das erfindungsgemäße Molekül (beziehungsweise der analogen Varianten) ist für die Anwendung bei Nierenerkrankungen verschiedener Ätiologie zur Therapie geeignet als Arzneimittel. Das erfindungsgemäße Arzneimittel bewirkt die Regeneration glomerulärer Podozyten. Das erfindungsgemäße Molekül kann weiter durch analytische Bestimmung der Blutkonzentration als Marker für die Krankheitsverläufe von Nierensyndromen benutzt werden und für Prognose und Prävention von der Niereninsuffizienz als ein wertvoller Faktor der Labormedizin angesehen werden.

## Beschreibung

Die vorliegende Erfindung betrifft ein Arzneimittel enthaltend ein Polypeptid, das zur Therapie von Nierenerkrankungen geeignet ist. Gegenstand der Erfindung ist auch ein *in-vitro* Verfahren zur Diagnose und zum Monitoring der Progredienz einer Nierenerkrankung durch Bestimmung des Ribonucleasegehaltes in Körperflüssigkeiten oder Geweben eines Patienten.

Ein akutes Nierenversagen kann jederzeit bei Fortschreiten in ein Terminalstadium übergehen, was ohne aufwendige Therapie wie Nierenersatzverfahren (Hämofiltration, Hämodialyse) zu einem vollständigen Funktionsverlust der Nieren führt. Das akute Nierenversagen (ANV) gehört zu häufigsten Todesursachen in den Industrienationen (über 10 %), wobei sich Entwicklungen besonders die bei Grunderkrankungen wie Diabetes mellitus Typ 2, Bluthochdruck aufgrund von Bewegungsmangel und Fehlernährung sowie Ursachen wie Einnahme von toxischen Stoffen (Rauchen, Alkohol, Medikamente) und weiter chronische, Entzündungen und Infektionen der Nieren, Verengungen der ableitenden Harnwege und angeborene Nierenkrankheiten in ANV übergehen oder in chronischem Nierenversagen (chronic kidney disease, CKD) enden.

Damit stellt die Diagnose der verschiedenen Nierenversagen, die Überwachung des Krankheitsverlaufes, Prophylaxe der Krankheitsformen und ihre spezifische Therapie eine außergewöhnliche Herausforderung (Unmet Need) dar.

Chronische Nierenerkrankungen (CKD) betreffen ca. 10% der westlichen Weltpopulation (Brück K., Stel V.S., Gambaro G. 2016; CKD Prevalence Varies across the European General Populations. JASN 27 (7) 2135 - 2147). Die Mehrheit der Nierenerkrankungen, die sich zu einer CKD weiterentwickeln beginnen im Glomerulus, der Filtrationseinheit der Niere. Dies ist eine Konsequenz aus der begrenzten Regenerationsfähigkeit des Glomerulus durch die limitierte Fähigkeit der glomerulären Podozyten zur Selbsterneuerung. Podozyten sind eine wesentliche Komponente der dreilagigen Filtrationsbarriere der Niere (Tryggvason, K: and Pettersson; E. 2003; Causes and consequences of proteinuria: the kidney filtration barrier and progressive renal failure. J Intern Med 254, 216 - 224). Die Funktion der Podozyten wird durch eine hochspezialisierte Zellfunktion, die Ausbildung einer signalaktiven Zell-Zell-Verbindung, der sogenannten Schlitzmembran, reguliert. Diese modifizierten Aherence-Junction bildet sich zwischen den podozytären Fußfortsätzen aus. Die Fußfortsätze sind spezialisierte Zellausläufer, mit feinregulierten Zytoskelettstrukturen die einem stetischen Auf- und Abbau unterliegen und damit den Auf- und Abbau der zellulären Fokalkontakte regulieren (Sever S and Schifer M. Actin dynamics at focal adhesions: a common endpoint and putative therapeutic target for proteinuric kidney diseases Kidney Int. 2018 Jun;93(6):1298-1307) Das Zytoskelett der Podozyten wird durch verschieden Proteine aufgebaut und. Es ist die Grundvoraussetzung für die Stabilisierung und den Aufbau der Schlitzmembran. Wird dieses Zytoskelett beispielsweise durch Mutationen oder Toxine beeinflusst, wirkt sich das auf die Funktionalität der Schlitzmembran aus, was in fast allen Fällen zu einer übermäßigen Ausscheidung von Proteinen über den Urin führt (Proteinurie). Für solche "Podozytopathien" stehen bisher keine spezifischen Therapieoptionen zur Verfügung. Im Allgemeinen werden diese Erkrankungen mit immunsuppressiven Medikamenten therapiert. Ob diese Therapie spezifische Effekte an den Podozyten induziert oder über andere Mechanismen sekundär positive Effekte triggert (möglichweise über die Induktion von Ribonucleasen) bleibt bisher offen.

Generell stellt die Proteinurie ein erhebliches Mortalitäts und Morbiditätsrisiko dar. Personen mit einer vermehrten Ausscheidung von Proteinen, wie Albumin, über den Urin (Albuminurie), haben ein erhöhtes Risiko, im weiteren Verlauf einen fortschreitenden Verlust der Nierenfunktion bis hin zum dialysepflichtigen Nierenversagen zu erleiden. Bei noch gegebener Nierenfunktion steigt allerdings mit zunehmender Eiweißausscheidung trotzdem die Mortalität sowie das Risiko, einen Herzinfarkt zu erleiden. Somit stellt die Proteinurie einen unabhängigen Risikofaktor für Herz-Kreislauferkrankungen dar.

Ausdifferenzierte Podozyten sind nach Abschluss der Organogenese nicht mehr zu einer regenerativen, mitotischen Zellteilung befähigt (Rennke, H.G. 1994; How does glomerular epithelial cell injury contribute to progressive glomerular damage? Kidney Int Suppl 45, S58-63). In der Erkrankungssituation gehen zunächst die Zell-Zellkontakte verloren und die Schlitzmembranproteine sind nur noch vermindert nachweisbar. Solange die Zellen noch vorhanden sind, besteht bei der richtigen Therapie noch ein Potential diese Zellverbindungen wiederaufzubauen. Bei länger anhaltenden Erkrankungszuständen sterben die Podozyten allerdings ab und mit ihnen geht dann der gesamte Glomerulus und das nachfolgende Nephron zu Grunde.

Ein der Erfindung zu Grunde liegendes Problem besteht mithin darin, eine Verbindung bereitzustellen, die in der Lage ist, die Differenzierung und Rekonstruktion der Podozyten wiederherzustellen, die Regeneration und Ausbildung der zellulären Fußfortsätze zu fördern und damit verbundene Nierenerkrankungen zu lindern oder zu heilen.

Es wurde überraschenderweise gefunden, dass das im erfindungsgemäßen Arzneimittel vorliegende Polypeptid als Modulator bei gestörtem Zytoskelettaufbau der Podozyten, besonders bei Differenzierungsstörungen, dienen kann.

Erfindungsgemäß kann das Polypeptid als Arzneimittel bei der Behandlung von Erkrankungen der Nierenfunktion eingesetzt werden, die durch Differenzierungsstörungen und Rekonstruktion der Podozytenstruktur und deren Proliferation verursacht werden. Dies trifft auf alle Nierenerkrankungen zu, die mit einer Eiweißausscheidung einhergehen.

Neben dem vollständigen Polypeptid Ribonuclease sind auch dessen Derivate, Varianten und Fragmente als Arzneimittel geeignet, sofern sie eine der nativen Ribonuclease vergleichbare Aktivität der Regeneration von Podocyten im krankheitsfall aufweisen.

Das erfindungsgemäße Arzneimittel kann insbesondere humane Pankreas-Ribonuclease, zum Beispiel humane Pankreas Ribonuclease 1 enthalten.

In einer Ausführungsform des erfindungsgemäßen Arzneimittels kann dieses eine Ribonuclease 1 enthalten, die mit dem Polypeptid der SEQ ID No 1 eine mindestens 70%ige, insbesondere mindestens 80%ige, insbesondere mindestens 90%ige, insbesondere mindestens 95%ige Sequenzidentität aufweist.

Das erfindungsgemäße Arzneimittel kann in einer im wesentlichen wässrigen Lösung, insbesondere in wässriger Lösung mit pharmazeutischen Hilfstoffen, vorliegen.

Insbesondere kann das erfindungsgemäße Arzneimittel Ribonuclease in einer zur parenteralen Verabreichung geeigneten Formulierung enthalten oder selbst als parenteral zu verabreichende Formulierung vorliegen.

Das erfindungsgemäße Arzneimittel kann die Ribonuclease vorzugsweise in einer Menge von mindestens einer funktionellen Unit mit Ribunucleaseaktivität pro Darreichungseinheit enthalten.

Gegenstand der Erfindung ist auch eine Ribonuclease zur Behandlung von Nierenerkrankungen, wobei die Ribonuclease vorzugsweise humane Pankreas-Ribonuclease ist. Die Ribonuclease weist vorzugsweise mit dem Polypeptid der SEQ ID No 1 eine mindestens 70%ige, insbesondere mindestens 80%ige, insbesondere mindestens 90%ige, insbesondere mindestens 95%ige Sequenzidentität auf.

Die erfindungsgemäß zu verwendende Ribonuclease fördert die Regeneration glomerulärer Podozyten und ist daher zur Behandlung von Nierenerkrankungen besonders geeignet.

Gegenstand der vorliegenden Erfindung ist auch die Verwendung von Pankreas Ribonuclease, insbesondere humaner Pankreas Ribonuclease oder hRNase 1 zur Identifizierung von Ribonucleaserezeptoren renaler Zellen in einem Assay. Entsprechende Assay Verfahren zur Identifizierung von Targets von Liganden sind dem Fachmann bekannt. Dabei können insbesondere markierte Pankreas Ribonucleasen eingesetzt werden.

Gegenstand der vorliegenden Erfindung ist darüber hinaus auch die Verwendung von Pankreas Ribonuclease, insbesondere humaner Pankreas Ribonuclease oder hRNase 1 zur Identifizierung von Substraten der Pankreas Ribonuclease, die mit der Degeneration von Podocyten in einem kausalen Kontext stehen. Entsprechende Screeningverfahren sind dem Fachmann geläufig.

Des weiteren ist Gegenstand der vorliegenden Erfindung ein *ex vivo* Verfahren zur Entfernung von Substraten der Pankreas Ribonuclease, die mit der Degeneration von Podozyten in einem kausalen Kontext stehen, insbesondere extrakorporalen Verfahren wie Hämodialyse, Hämofiltration, Hämodiafiltration, Peritonealdialyse, Plasmaaustauschbehandlung, bzw. Behandlung mit unspezifischen Filtern oder spezifischen Ribonuclease enthaltenden Filtereinheiten, die die Substrate aus dem Blut entfernen bzw. inaktivieren.

Gegenstand der Erfindung ist auch ein *in vitro* Diagnostikverfahren, das die mengenmäßige Anwesenheit des erfindungsgemäß eingesetzten Polypeptids, insbesondere Ribonuclease, zum Beispiel humane Ribonuclease, als Indikator für den Schweregrad der Nierendegeneration oder die Reversibilität der Nierenfunktion nutzt. Die Bestimmung der Konzentration der Ribonuclease im Verlaufe der Erkrankung ist unter anderem für die Betrachtung der Krankheitsrelevanz wichtig. Die Ribonuclease, insbesondere humane Pankreas Ribonuclease, zum Beispiel humane Pankreas Ribonuclease 1 ist mittels verschiedener Analyse-Methoden der Peptidchemie messbar, zum Beispiel:
1. Immunchemisch durch Techniken wie ELISA etc.
2. Verschiedene Techniken der Gelchromatographie,
3. Kapillarelektrophorese, Dünnschicht-Chromatographie
4. HPLC Darstellung mit Reverse-Phase oder Affinitätschromatographie
5. quantitative Massen-Spektrometrie LC-MS oder Quadrupol, usw.

Fig. 1: Phänotyp des PKCε-knockouts.
Fig. 2: hRNase1 enthaltende Fraktionen verbessern die Dysregulation des Aktin-Zytoskelettes in PKCε-defizierten Podozyten.
Fig. 3: Darstellung des Rescue-Effekts rekombinanter humaner RNAse 1 Einwirkung auf die Zellstruktur der PKCε-defizierten Podzyten.

Die Erfindung wird im Folgenden näher erläutert.

Ribonukleasen sind zur Gruppe III der EC-Klassifikation der Enzyme gehörige Hydrolasen, die innerhalb eines RNA-Moleküls die Esterspaltung zwischen der 5'-Phosphatgruppe eines Nukleotids und der 3'-Hydroxylgruppe eines benachbarten hydrolytisch spalten. Man kann dabei zwei Klassen von Ribonukleasen unterscheiden.

RNase I (Pankreas-RNase): Spaltung nur hinter Pyrimidinnukleotiden, die RNase I ist durch Heparin hemmbar.

RNase II (Pflanzliche RNase): Spaltung hinter Purin- und PyrimidinnukleotidenDie vorliegende Erfindung beruht auf der überraschenden Entdeckung, dass das Polypeptid als Modulator bei gestörtem Cytoskelettaufbau der Podozyten, besonders bei Differenzierungsstörungen, dient. Das Polypeptid kann als Arzneimittel bei der Behandlung von Erkrankungen der Nierenfunktion eingesetzt werden, die durch Differenzierungsstörungen und Rekonstruktion der Podozytenstruktur und deren Proliferation verursacht werden. In der wissenschaftlichen Literatur wird auch eine Funktion der Ribonucleasefamilie als Rezeptor Ligand diskutiert (Wang et al. Journal of Biomedical Science(2018) 25:83). So wird dort hRNase 5 als Liganden des EGFR RTK beschrieben damit ist eine Bindung der Mitglieder der RNase Superfamilie an Proteine der Rezeptor Tyrosinkinasenfamilie (RTK oder RYK) wahrscheinlich.

Podozyten sind hochspezialisierte Zellen, die in den Nierenkörperchen wesentlich an der Filtration des Blutes beteiligt sind. Diese Zellen sind terminal differenziert, d.h. sie können sich nicht mehr teilen und so regenerieren oder geschädigte Podozyten ersetzen. Bei den meisten Nierenerkrankungen beobachtet man einen fortschreitenden Podozytenverlust und das diese Zellen trotz ihrer terminalen Differenziertheit wieder in den Zellzyklus eintreten, diesen jedoch nicht bis zur Zellteilung durchlaufen können und stattdessen zu Grunde gehen.

Die Wirkung des Polypeptids der vorliegenden Erfindung kann mit einem Bioassay nachgewiesen werden. Der entsprechende Assay wird in den Beispielen näher erläutert.

Das erfindungsgemäße Arzneimittel enthält insbesondere humane Pankreas-Ribonuclease. Die Aminosäuresequenz des im erfindungsgemäßen Arzneimittel enthaltene Polypeptids der humanen Pankreas-Ribonuclease, inklusive des Signalpeptids ist gemäß Eintrag in der Datenbank uniprot wie folgt https://www.uniprot.org/uniprot/P07998#sequences (SEQ ID No. 2):

MALEKSLVRLLLLVLILLVLGWVQPSLGKESRAKKFQRQMDSDSSPSSSSTYCNQMM-
RRRNMTQGRCKPVNTFVHEPLVDVQNVCFQEKVTCKNGQGNCYKSNSSMHITDCRLTNGSRY
PNCAYRTSPKERHIIVACEGSPYVPVHFDASVEDST

Unterstrichen sind drei potenzielle N-Glykosylierungsstellen, von welchen zwei besetzt sind.

Die kursiv wiedergegebenen Aminosäuren in obiger Sequenz stellen das Signalpeptid dar.
MALEKSLVRLLLLVLILLVLGWVQPSLG (SEQID No3)

Das wirksame Polypeptid ist (SEQ ID No. 1):

KESRAKKFQRQMDSDSSPSSSSTYCNQMMRRRNMTQGRCK-
PVNTFVHEPLVDVQNVCFQEKVTCKNGQGNCYKSNSSMHITDCRLTNGSRYPNCAYRTSPKE
RHIIVACEGSPYVPVHFDASVEDST

Die Ribonuclease wurde ursprünglich in Hämofiltrat Fraktionen als wirksames Agens zur Regeneration von Podozyten in einem Bioassay identifiziert. Zwar ist eine Isolierung der humanen Pankreas Ribonuclease aus Hämofiltrat grundsätzlich möglich, allerdings ist die Gewinnung des Polypeptids auch durch Totalsynthese nach Merrifield möglich. Vorzugsweise wird das Polypeptid jedoch durch rekombinante Methoden und Expression in geeigneten Systemen bereitgestellt. Dabei empfiehlt sich die Expression der entsprechenden Polynucleotide, die für die Pankreas Ribonuclease kodieren, in biologischen Systemen, insbesondere eukaryotischen Zellen, die das exprimierte Polypeptid posttranslatorisch mit dem humanen Glykosylierungsmuster versehen. Je nach verwendeter Zellkultur zur Expression des Polypeptids können aber auch andere Säugetier Pankreas Ribonucleasen, die aus Zellen von Säugetieren exprimiert werden, eingesetzt werden, beispielsweise solche aus Primaten wie Affen, Schimpansen, Makaken und Gorillas aber auch von Nichtprimaten wie Mäusen, Ratten, Meerschweinchen, Hamstern, Kaninchen, Kühen, Pferden, Schafen, Ziegen und Schweinen.

Pankreas Ribonuclease Polypeptide sind im erfindungsgemäßen Sinne auch solche, die funktionale Äquivalente zur humanen Pankreas Ribonuclease darstellen, d. h. eine ähnliche Aminosäuresequenz aufweisen und in der Lage sind als Modulator der Podozyten zu wirken

In einer Ausführungsform des erfindungsgemäßen Arzneimittels kann dieses eine Ribonuclease enthalten, die mit dem Polypeptid der SEQ ID No. 1 eine mindestens 70%ige, insbesondere mindestens 80%ige, insbesondere mindestens 90%ige, insbesondere mindestens 95%ige Sequenzidentität aufweist.

In einer anderen Ausführungsform des Polypeptids zur Verwendung als Arzneimittel kann dieses auch eine Variante der Pankreas Ribonuclease sein. Varianten sind Fragmente des Polypeptids welche typischerweise mindestens 5-20 flankierende Aminosäuren auf beiden Seiten der auf die Regeneration der Podozyten wirkenden Bereiche aufweisen. Fragmente im Sinne der Erfindung umfassen typischerweise mindestens 25, 50, 75, 100 oder 150 Aminosäuren.

Pankreas Ribonuclease Varianten umfassen aber auch Polypeptide, die im Wesentlichen identisch mit der nativen Aminosäuresequenz der Pankreas Ribonuclease sind. Diese Varianten umfassen auch Polypeptide mit Änderungen der Aminosäuresequenz des nativen Polypeptids wie die Deletionen, Insertionen und oder Substitutionen.

Der Begriff "Deletion" bezieht sich dabei auf die Abwesenheit einer oder mehrere Aminosäurereste im Polypeptid. Der Begriff "Insertion" bedeutet die Hinzufügung einer oder mehrerer Aminosäuren in dem betreffenden Polypeptid. Unter "Substitution" wird die Ersetzung einer oder mehrerer Aminosäurereste durch andere Aminosäuren in dem Polypeptid verstanden. Typischerweise sind solche Änderungen konservativer Natur wodurch die Aktivität des Varianten Polypeptids im Vergleich zum nativen Polypeptid im Wesentlichen ähnlich oder gleichbleibt. Im Falle einer Substitution weist die Aminosäure, welche die andere Aminosäure im nativen Polypeptid ersetzt, üblicherweise ähnliche strukturelle und oder chemische Eigenschaften auf. Insertionen und Deletionen betreffen üblicherweise Bereiche von 1-5 Aminosäuren, obwohl dies abhängig ist von der Lokalisierung der Insertion, sodass auch noch mehr Aminosäuren eingefügt oder entfernt werden können ohne die Funktion des nativen Polypeptids ungebührlich zu verändern. Variationen des nativen Polypeptids können insbesondere auf der Polynucleotide Ebene eingeführt werden, beispielsweise durch die wohl etablierten Methoden der stellenspezifischen Mutagenese [site-directed mutagenesis (Carter, et al. (1986) Nucl. Acids Res. 13:4331; Zoller et al. (1987) Nucl. Acids Res. 10: 6487), cassette mutagenesis (Wells et al. (1985) Gene 34: 315), restriction selection mutagenesis (Wells, et al. (1986) Philos. Trans. R. Soc. London SerA 317: 415), and PCR mutagenesis (Sambrook, et al. (1989) Molecular Cloning, Cold Spring Harbor Laboratory Press)].

Pankreas Ribonuclease Varianten umfassen auch modifizierte Formen des Polypeptids wie auch dessen Derivate. Modifizierte Pankreas Ribonuclease sind Polypeptide, bei denen eine oder mehrere Aminosäuren der nativen Sequenz so geändert wurden, dass ein nicht natürlich vorkommender Aminosäurerest in der Polypeptidkette vorhanden ist. Solche Modifikationen werden grundsätzlich während oder nach der Proteintranslation durchgeführt und beinhalten beispielsweise Phosphorylierung, Glykosylierung, Sulfonierung, Vernetzung, Acylierung, Hesylierung, PEGylierung oder auch proteolytische Spaltung. Durch die genannten Modifizierungen erhaltenen Polypeptide, die auch Fragmente des erfindungsgemäß zu verwendenden Polypeptid Ribonuclease sein können, werden auch als Derivate bezeichnet.,

Typischerweise können die Austausche von Aminosäuren in konservativer Weise erfolgen. Unter einem konservativen Austausch wird eine Mutation verstanden, bei der ein Codon durch ein anderes ersetzt wird. Dabei wird eine andere Aminosäure codiert, die jedoch chemisch mit der ursprünglichen Aminosäure verwandt ist, beispielsweise Glycin mit Alanin oder Threonin mit Serin. Ein nicht-konservativer Austausch liegt hingegen vor, wenn an die Stelle des ursprünglichen Codons ein Codon tritt, das eine Aminosäure mit anderen chemischen Eigenschaften codiert, beispielsweise Glycin mit Lysin.

Die Begriffe "identisch" oder Prozent "identisch" im Zusammenhang mit zwei oder mehr Polypeptiden bezieht sich auf zwei oder mehr Sequenzen oder Untersequenzen, die gleich oder einen bestimmten Prozentsatz von Aminosäuren die identisch sind aufweisen, wenn diese, unter Verwendung eines Sequenzvergleichsalgorithmus, auf maximale Übereinstimmung verglichen und aligned werden. Optimales Alignment zum Vergleich von Sequenzen kann beispielsweise mittels der folgenden Algorithmen durchgeführt werden: Lokales Homology Alignment von Smith &Waterman, Adv. Appl. Math. 2: 482 (1981), Homology Alignment Algorithmus von Needleman & Wunsch, J. Mol.Biol. 48: 443 (1970), Pearson & Lipman, Proc. Nat'l. Acad.Sci. USA 85: 2444 (1988), (GAP, BESTFIT, FASTA, und TFASTA in Wisconsin Genetics Software Package, Genetics Computer Group, 575 Science Dr., Madison, WI), aber auch visueller Untersuchung [vergleiche, Current Protocols in Molecular Biology, (Ausubel, F. M. et al., eds.) John Wiley & Sons, Inc., New York (1987-1999, einschließlich Supplement 46 (April 1999)].

Der Ausdruck "im Wesentlichen identisch" o. ä. Ausdrücke, die im Zusammenhang mit zwei Polypeptiden verwendet werden bezieht sich auf zwei oder mehr Sequenzen oder Untersequenzen, die mindestens eine 70%ige, insbesondere mindestens 80%ige, insbesondere mindestens 90%ige, insbesondere mindestens 95%ige Sequenzidentität aufweisen, wenn diese, unter Verwendung eines Sequenzvergleichsalgorithmus, auf maximale Übereinstimmung verglichen und aligned werden. Substantielle Identität existiert insbesondere dann vor, wenn ein Sequenzbereich, der mindestens eine Länge von 40-60 Aminosäuren, insbesondere 60-80 Aminosäuren, insbesondere mehr als 90-100 Aminosäurereste aufweist und insbesondere im Wesentlichen identisch über die gesamte Länge der Aminosäuresequenz des nativen Polypeptids ist.

Das erfindungsgemäße Arzneimittel kann nach in einer im Wesentlichen wässrigen Lösung, insbesondere in wässriger Lösung mit pharmazeutischen Hilfstoffen vorliegen.

Die Hilfsstoffe können einzelne oder in Kombinationen nicht nur zum Polypeptid zugegeben werden, sondern auch zu einer finalen Formulierung. Die Hilfsstoffe können an verschiedenen Stellen der galenischen Zubereitung des Arzneimittels zugefügt werden.

Es kann sich empfehlen das Polypeptid gegen Aggregation oder Oligomerisation zu stabilisieren. Nötigenfalls kann auch ein bakteriostatisches Agens, wie Benzylalkohol, ein oberflächenaktives Material, wie Tween 20, ein isotonisches Mittel, wie Mannitol, eine oder mehrere stabilisierende Aminosäuren, wie Lysin oder Arginin sowie ein Antioxidans der Formulierung beigefügt werden.

Insbesondere enthält das erfindungsgemäße Arzneimittel Ribonuclease in einer zur parenteralen Verabreichung geeigneten Formulierung.

Das erfindungsgemäße Arzneimittel enthält die Ribonuclease vorzugsweise in einer Menge von mindestens einem funktionellen Molekül pro Darreichungseinheit.

Gegenstand der Erfindung ist auch eine Ribonuclease zur Behandlung von Nierenerkrankungen, wobei die Ribonuclease vorzugsweise humane Pankreas-Ribonuclease ist. Die Ribonuclease weist vorzugsweise mit dem Polypeptid der SEQ ID No 1 eine mindestens 70%ige, insbesondere mindestens 80%ige, insbesondere mindestens 90%ige, insbesondere mindestens 95%ige Sequenzidentität auf.

Die erfindungsgemäß zu verwendende Ribonuclease fördert die Regeneration glomerulärer Podozyten und ist daher zur Behandlung von Nierenerkrankungen besonders geeignet.

Fragmente der Ribonuclease, die im erfindungsgemäßen Arzneimittel enthalten sein können, werden typischerweise durch Spaltung der Polypeptidkette der Ribonuclease erhalten. Verfahren zur Fragmentierung durch Spaltung der Polypeptidkette sind dem Fachmann bekannt.

So lassen sich Polypeptide durch Enzyme, sogenannte Proteasen, spalten. Je nachdem, ob die Proteasen Polypeptide (Proteine) innerhalb der Aminosäurekette spalten oder am Ende, unterscheidet man zum einen Proteinasen (auch Endopeptidasen genannt). Sie spalten Proteine innerhalb der Aminosäurekette. Meist erkennen sie spezifische Sequenzabschnitte innerhalb eines Proteins, an denen sie angreifen können, und spalten dort.

Zum anderen spalten Exoproteasen (auch Exopeptidasen genannt) einzelne Aminosäuren von den Enden des Proteins ab. Im Gegensatz zu den Proteinasen zerlegen sie vor allem kleinere Peptide und keine Proteine. Man unterscheidet:
Carboxypeptidasen, die die Aminosäuren vom Carboxylende (C-Terminus) her abspalten sowie Aminopeptidasen, die die Aminosäuren vom Aminoende (N-Terminus) her abspalten.

Spezifische Proteasen spalten die Peptidbindung zwischen zwei bestimmten Aminosäuren; in manchen Fällen erkennen sie auch mehr als nur ein Substrat. Diese Proteasen werden eingesetzt, um z.B. die Verwandtschaft von Proteinen zu untersuchen, Proteine für die Sequenzierung vorzubereiten oder um aktive Domänen eines Proteins zu isolieren. Einige Beispiele für solche Proteasen zeigt die Tabelle:

**Tab.1**

| Spezifische Proteasen und ihre Eigenschaften | |
|---|---|
| Protease | Spezifität der Spaltung |
| Pepsin | Phe⇓X, Leu⇓-X und Paare unpolarer Aminosäuren |
| Trypsin | Arg⇓-X, Lys⇓-X |
| Chymotrypsin | Tyr⇓-X, Phe⇓X, Trp⇓-X |
| Thrombin | Arg⇓-X |
| Thermolysin | X⇓-Leu, X⇓-Phe, andere unpolare Reste |
| Endoproteinase Lys-C | Lys⇓-X |
| Endoproteinase Glu-C | Glu⇓-X (z.T. auch Asp⇓X) |
| Endoproteinase Arg-C (Clostripain) | Arg⇓-X |

Im Gegensatz zu den spezifischen Proteasen spalten unspezifische Proteasen die Peptidkette vor oder nach einer ganzen Reihe von Aminosäuren und generieren damit sehr viel kleinere Spaltstücke. Einige Beispiele für solche unspezifischen Proteasen zeigt die Tabelle:

**Tab.2**

| Unspezifische Proteasen und ihre Eigenschaften | |
|---|---|
| Protease | Spezifität der Spaltung |
| alkalische Protease | Phe⇓X, Leu⇓-X, Val⇓-X, Ile⇓-X, Trp⇓X, Tyr⇓-X |
| Papain | Arg⇓X, Lys⇓X, Phe⇓-X |
| Proteinase K | breites Substratspektrum |

Zur proteolytischen Analyse von Peptiden können neben Proteasen auch bestimmte Chemikalien eingesetzt werden. Bromcyan (BrCN) ist das wichtigste Reagenz dieser Art und spaltet Proteine auf der C-terminalen Seite von MethioninResten. Dabei entsteht Peptidyl-homoserinlacton. Die resultierenden Peptid-Fragmente lassen sich dann im Polyacrylamid-Gel auftrennen und durch eine Färbung visualisieren.

**Tab.3**

| Typische Reagenzien für die chemische Proteolyse und ihre Eigenschaften | |
|---|---|
| Reagenz | Spezifität der Spaltung |
| Bromcyan | Met⇓-X |
| partielle Säurehydrolyse | Asp⇓-Pro |
| Hydroxylamin | Asn⇓-Gly |

Ein Sonderfall der Proteolyse ist die so genannte limitierte Proteolyse. Im Gegensatz zur Totalhydrolyse ist der Proteinverdau bei dieser Methode nicht vollständig, sondern erfolgt unter genau definierten Reaktionsbedingungen (die Proteolyse verläuft also limitiert). In den globulären Bereichen eines nativen Proteins sind die Peptidbindungen weitaus weniger exponiert als die Peptidbindungen an der Oberfläche des Proteins. Das bedeutet, dass bei kurzer Einwirkzeit einer Protease oder bei sehr geringer Protease-Konzentration unter Umständen zunächst die einzelnen Proteindomänen getrennt werden, bevor die Protease auch weiter innen liegende Spaltstellen erreicht. Die limitierte Proteolyse wird also mit einer Protease-Verdünnung und/oder suboptimalen Reaktionsbedingungen durchgeführt und nach kurzer Zeit gestoppt. Bei dieser Methode wird das Protein in seiner nativen Form (und nicht nach einer Denaturierung) proteolytisch gespalten; dieses hat mitunter Konsequenzen für die Wahl der Protease. Benötigt das Protein z.B. EDTA für optimale Stabilität, kann für den Verdau keine Metalloprotease verwendet werden.

Weitere Informationen zur Proteinanalytik und Verfahrensweise zur proteolytischen Spaltung von Polypeptiden sind dem Lehrbuch "Arbeitsmethoden der Biochemie" von Alfred Pingoud, Claus Urbanke, Walter de Gruvter, zum Beispiel Kapitel 5.1.6 entnehmbar.

Die Nierenerkrankung kann insbesondere ausgewählt sein aus der Gruppe bestehend aus: diabetischer Nephropathie, nephrotischem Syndrome, nephritischem Syndrome, toxischen Nebenwirkungen anderer Therapien, glomerulären Erkrankungen, wie membranöse Glomerulonephritis, focal segmentale Glomerulosklerose (FSGS), IgA Nephropathie, IgM Nephropathie, Membranoproliferative Glomerulonephritis, Minimal change disease, hypertensive Nephrosklerose, und interstitielle Nephritis, Morbus Fabry, Infektionen, Aminoacidurien, Fanconi syndrome, Schwermetallvergiftungen, Sichelzellkrankheit, Hämoglobinurie, Multiplem Myelom, Myoglobinurie, Organabstoßung, insbesondere Nierentransplantatabstoßung und Rezidiverkrankungen des Nierentransplantates, Präeklampsie, akutem Nierenversagen und genetischen Podozytopathien.

Eine Hauptfunktion der Ribonuclease besteht in der Spaltung von RNA Molekülen. Der Wirkungsmechanismus, der der Regeneration der Podozyten auf die Gabe von Ribonuclease zugrunde liegt, ist nicht bekannt. Ohne an eine Erklärung der Wirkung gebunden zu sein, kann ein durch die Ribonuclease, insbesondere humane Pankreas Ribonuclease 1, verursachter Abbau von RNA Molekülen, die mit der Ätiologie einer Nierenerkrankung, die mit der Degeneration von Podozyten verbunden ist, im Zusammenhang steht, dazu führen, dass eine Regeneration der Podozyten einsetzt. In Ergänzung oder sogar anstelle der Gabe des erfindungsgemäßen Arzneimittels könnte auch eine Entfernung der durch die Ribonuclease abgebauten RNA zur Regeneration der Podozyten beitragen. Gegenstand der vorliegenden Erfindung ist mithin auch eine in-vitro durchgeführte Entfernung von nicht abgebauter RNA, insbesondere ein in vitro Verfahren zur Entfernung von Substraten der Pankreas Ribonuclease, die mit der Degeneration von Podozyten in einem kausalen Kontext stehen. Als entsprechende Verfahren kommen insbesondere extrakorporale Verfahren wie Hämodialyse, Hämofiltration, Hämodiafiltration, Peritonealdialyse, Zytosorb- und andere unspezifische Filter, sowie Plasmaaustauschbehandlungen infrage. In einer Ausführungsform des erfindungsgemäßen extrakorporalen Verfahrens kann die zu reinigenden Körperflüssigkeit nach deren Entnahme mit an einem Träger mobilisierten Ribonuclease in Kontakt gebracht werden. Dabei werden die mit der Ätiologie der Nierenerkrankung im Zusammenhang stehenden Ribonucleinsäuren abgebaut. In einer anderen Ausführungsform können die RNA-Bruchstücke aus der Körperflüssigkeit durch Extrakorporalverfahren entfernt werden.

Ein anderer Gegenstand der vorliegenden Erfindung ergibt sich aus der Diskussion in der wissenschaftlichen Literatur, dass Ribonuclease als Liganden für Rezeptoren infrage kommen. Insbesondere die Bindung der Ribonuclease an Rezeptor Tyrosinkinasen spricht für diese Annahme. Durch die Bindung der Ribonuclease insbesondere der humanen Pankreas Ribonuclease 1 an ein entsprechendes Target, wahrscheinlich ein Rezeptor aus der Familie der Rezeptor Tyrosinkinasen (RTK), wird eine Signalkaskade ausgelöst, in deren Folge Podozyten regenerieren.

Mittels des der Erfindung zugrunde liegenden Befundes, dass Ribonuclease, insbesondere humane Ribonuclease 1 die Regeneration von Podozyten bewirken kann, lassen sich auch die entsprechenden Targets der Ribonucleases zielgerichtet aufspüren. Mit dem Fachmann einschlägig bekannten Bioassays lassen sich die Targets (Rezeptoren) der Ribonuclease, insbesondere humanen Ribonuclease, wie hRNase1, zum Beispiel durch Bindungsexperimente mit markierten Ribonucleasen, insbesondere markierter hRNase1, identifizieren.

Gegenstand der vorliegenden Erfindung ist mithin auch die Verwendung von Pankreas Ribonuclease, insbesondere humaner Pankreas Ribonuclease oder hRNase 1 zur Identifizierung von Ribonucleaserezeptoren in einem Assay. In einer Ausführungsform können dabei markierte Ribonucleasen (zum Beispiel radioaktiv oder Fluoreszenz) eingesetzt werden.

Die Identifizierung der entsprechenden Targets oder Rezeptoren der Ribonuclease insbesondere humanen Pankreas Ribonucleasen kann von therapeutisch hohen Nutzen sein, da bei Kenntnis des Rezeptors dann eine Entwicklung von niedermolekularen Stoffen gezielt ermöglicht wird, welche die Wirkung des hochmolekularen Proteins Ribonuclease simulieren, therapeutisch leichter eingesetzt werden können als die Proteine selbst. Dabei können sowohl antagonistische wirkender Substanzen als auch agonistisch wirkende Substanzen identifiziert werden.

Gegenstand der vorliegenden Erfindung ist mithin auch die Verwendung von Pankreas Ribonuclease, insbesondere humaner Pankreas Ribonuclease oder hRNase 1 zur Identifizierung von Substraten der Pankreas Ribonuclease, die mit der Degeneration von Podozyten in einem kausalen Kontext stehen.

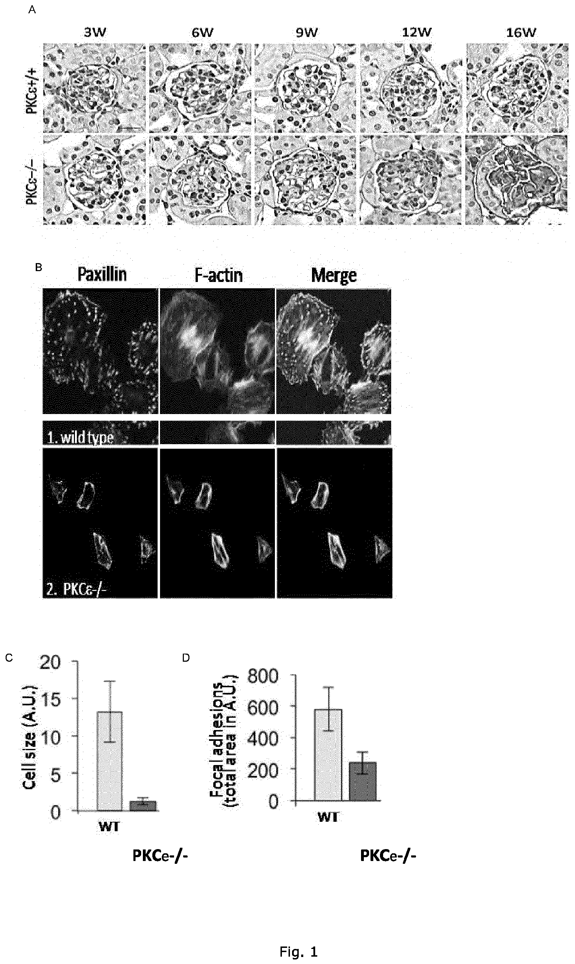

Fig. 1: Phänotyp des PKCε-knockouts. PKCε-Defizit verursacht Glomerulosklerose in Mäusen und Dysregulation des Aktin-Zytoskelettes in murinen Podozyten.
   A) Es entwickelt sich ab der 12. Woche in PKCε-defizierten Mäusen eine schwere Glomelurosklerose und Proteinurie.
   B) Mit der Paxillin Immunofluoreszenz-Färbung sind die fokalen Adhäsionsmolekülen in Podozyten sichtbar. F-Aktin ist visualisiert durch Phalloidin-Färbung. Die Aktin-Zytoskeletten sind dysreguliert in den PKCe-defizierten Podozyten.
   C) Mit Image J wurde die Zellgröße analysiert. Die Zellgröße der PKCε-defizierten Podozyten ist viel kleiner als die von den Wildtyp-Podozyten.
   D) Die Zahl der fokalen Adäsionsmolekülen in PKCε defizierten Podozyten ist auch deutlich kleiner als die in Zahl in Wildtyp-Podozyten.

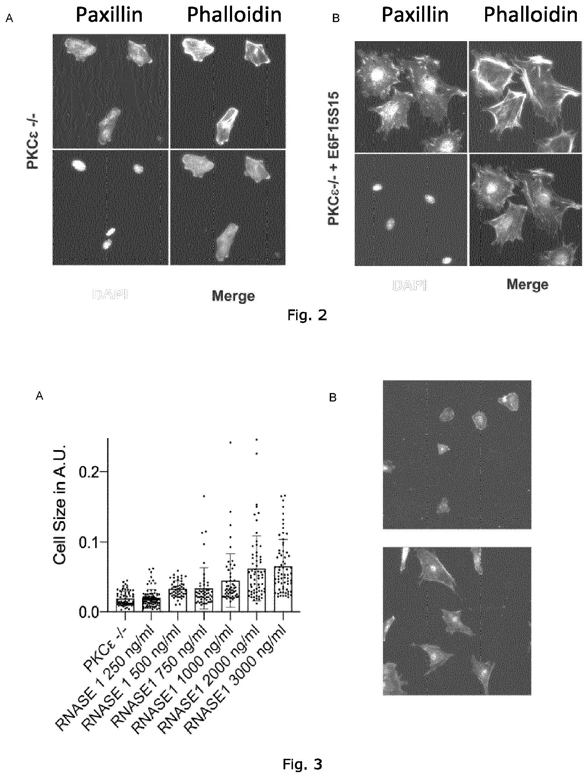

Fig. 2: hRNase1 enthaltende Fraktionen verbessern die Dysregulation des Aktin-Zytoskelettes in PKCε-defizierten Podozyten. Die PKCε-defizierte Podozyten sind mit unterschiedlichen aus Hämofiltrat isolierten Fraktionen, die hRNase1 enthalten, behandelt. Nach 24-stündiger Inkubation werden die Podozyten fixiert und mit Immunfluoreszenz gefärbt. A) PKCε-defizierte Podozyten zeigen den Phänotyp mit kleiner Zellgröße und dysreguliertem Aktin-Zytoskelett. B) Die fraction E6F15S ist in der Lage den Phänotyp zu verbessern. Sowohl die fokalen Adhäsionsmoleküle als auch das Aktin-Zytoskelett ist sehr gut darstellbar.
Fig. 3: Rekombinate humane RNAse 1 hat einen Rescue-Effekt auf die Zellstruktur der PKCε-defizierte Podzyten. Die PKCε-defizierte Podozyten sind mit rekombinanter humaner RNAse in unterschiedlichen Konzentration für 24 Studen inkubiert. Der Rescue-Effekt ist abhängig von der Konzentration der RNAse1. Schon ab 250 ng/ml ist ein leichter Rescue-Effekt auf die PKCε-defizierten Podozyten darstellbar. A) Mit gesteigerter Konzentration ist der Rescue-Effekt zu sehen und bis 3000 ng/ml erreicht der Effekt sein Maximum. B) Die Immunfluoreszenz-Färbung zeigt den Rescue-Effekt mit gesteigerter Zellgröße und Zunahme der Fokalkontaktdichte (oben ohne RNAse, unten mit RNAse.

### Beispiele

### Bioassay

### Bestimmung der Wirkung von Ribonuclease auf Podozyten Modifikation

Immortalisierte PKCε-/- Podozytes (SV129 background) wurden auf Coverslips in 24 Well Platten ausgesät und für die Dauer von sieben Tagen vor der Behandlung differenziert. Rekombinante humane oder bovine Ribonuclease 1 wurde zu 400 µl Medium (RPMI 1046 Medium mit 10 % FCS und ein Prozent Penicillin/Streptavidin) in jedes Well hinzugefügt und für 24 Stunden bei 37 °C inkubiert.

Die so behandelten Podozyten wurden zuerst in 4 % PFA für 10-15 min fixiert, danach mit PBS gewaschen und in PBS bei 4 °C gelagert. Die Zellen wurden mit 0,1 % Triton X- 100 für eine Dauer von 10 Minuten permeabilisiert und mit PBS gewaschen. Die unspezifische Antikörper Bindung wurde mittels 10 % Eselserum für eine Dauer von 30 Minuten bei Raumtemperatur blockiert. Danach wurden die Zellen mit primärem Antikörper (Anti-Paxillin Antibody, clone 5H11, Cat. #05-417, Millipore) bei 4 °C über Nacht bzw. 1 Stunde bei Raumtemperatur inkubiert. Ungebundene primäre Antikörper wurde dreimal mit PBS gewaschen und mit korrespondierendem sekundärem Antikörper und Phalloidin (Alexa FluorTM 555 Phalloidin, Cat. #A34055, Invitrogen) für 1 Stunde bei Raumtemperatur inkubiert. Die Zellen wurden danach erneut dreimal für 5 Minuten mit PBS gewaschen. Die Coverslips wurden kurz mit bidestilliertem Wasser gewaschen, mit Aquapolymout Medium (mit DAPI) auf Objektträgern fixiert und bei 4 °C gelagert. Die Färbung wurde unter einem Leica DMLB Mikroskop mit einer Leica DFC425C Kamera oder unter einem Leica-2 konfokalem Mikroskop sichtbar gemacht.

## Patentansprüche

1. Arzneimittel enthaltend Ribonuclease und /oder ein Fragment, eine Mutante und/oder ein Derivat der Pankreas-Ribonuclease.

2. Arzneimittel nach Anspruch, wobei die Pankreas-Ribonuclease aus Säugetieren stammt, insbesondere humane, porcine oder bovine Pankreas-Ribonuklease ist.

3. Arzneimittel nach Anspruch 1, wobei die Ribonuclease humane Pankreas-Ribonuclease, insbesondere humane Pankreas Ribonuclease 1 (hRNase 1) ist.

4. Arzneimittel nach mindestens einem der Ansprüche 1 bis 3, wobei die Pankreas-Ribonuclease ein Fragment, eine Mutante und/oder ein Derivat der Pankreas-Ribonuclease, ist die jeweils eine Regeneration glomerulärer Podozyten bewirken.

5. Arzneimittel nach mindestens einem der Ansprüche 1 bis 4, wobei die Ribonuclease eine mit der SEQ ID No 1 zu mindestens 70%ige, insbesondere mindestens 80%ige, insbesondere mindestens 90%ige, insbesondere mindestens 95%ige Sequenzidentität aufweist.

6. Arzneimittel nach mindestens einem der Ansprüche 1 bis 5, wobei das Fragment der Ribonuclease durch Proteolyse mit Enzymen oder Chemikalien, wie Bromcyan oder Hydroxylamin.

7. Arzneimittel nach Anspruch 5 mit der SEQ ID No 1.

8. Arzneimittel nach mindestens einem der Ansprüche 1 bis 7 in im wesentlichen wässriger Lösung, insbesondere in wässriger Lösung mit pharmazeutischen Hilfstoffen.

9. Arzneimittel nach mindestens einem der Ansprüche 1 bis 8, wobei die Ribonuclease zur parenteralen Verabreichung formuliert ist.

10. Arzneimittel nach mindestens einem der Ansprüche 1 bis 9, wobei die Ribonuclease in einer Menge von mindestens einer funktionellen Moleküleinheit vorliegt.

11. Ribonuclease zur Behandlung von Nierenerkrankungen.

12. Ribonuclease zur Behandlung von Nierenerkrankungen nach Anspruch 11, wobei die Ribonuclease Pankreas-Ribonuclease ist, insbesondere humane, porcine oder bovine Pankreas-Ribonuclease ist.

13. Ribonuclease zur Behandlung von Nierenerkrankungen nach Anspruch 11 oder 12, wobei die Nierenerkrankung ausgewählt ist aus der Gruppe bestehend aus diabetischer Nephropathie, nephrotischer Syndrome, nephritischem Syndrome, toxische Nebenwirkungen anderer Therapien, glomerulärer Erkrankungen, wie membranöse Glomerulonephritis, Focal segmentale Glomerulosklerose (FSGS), IgA Nephropathie, IgM Nephropathie, Membranoproliferative Glomerulonephritis, Minimal change disease, hypertensiven Nephrosklerose, und interstitiellen Nephritis, Morbus Fabry, Infektionen, Aminoacidurie, Fanconi syndrome, Schwermetallvergiftung, Sichelzellkrankheit, Hämoglobinurie, Multiples Myelom, Myoglobinurie, Organabstoßung, Präeklampsie, akutem Nierenversagen und genetischen Podozytopathien.

14. Ribonuclease zur Behandlung von Nierenerkrankungen nach mindestens einem der Ansprüche 11 bis 13, wobei die Ribonuclease eine mit der SEQ ID No 1 zu mindestens 70%ige, insbesondere mindestens 80%ige, insbesondere mindestens 90%ige, insbesondere mindestens 95%ige Sequenzidentität aufweist.

15. Ribonuclease zur Behandlung von Nierenerkrankungen nach mindestens einem der Ansprüche 11 bis 14 mit der SEQ ID No 1.

16. Ribonuclease zur Behandlung von Nierenerkrankungen nach mindestens einem der Ansprüche 11 bis 15, wobei die Behandlung der Nierenerkrankung durch Regeneration glomerulärer Podozyten erfolgt.

17. Verwendung von Pankreas Ribonuclease, insbesondere humaner Pankreas Ribonuclease oder hRNase 1 zur Identifizierung von Ribonucleaserezeptoren renaler Zellen in einem Assay.

18. Verwendung gemäß Anspruch 17 wobei markierte Pankreas Ribonuclease eingesetzt werden.

19. Verwendung von Pankreas Ribonuclease, insbesondere humaner Pankreas Ribonuclease oder hRNase 1 zur Identifizierung von Substraten der Pankreas Ribonuclease, die mit der Degeneration von Podocyten in einem kausalen Kontext stehen.

20. *Ex-vivo* Verfahren zur Entfernung von Substraten der Pankreas Ribonuclease, die mit der Degeneration von Podocyten in einem kausalen Kontext stehen, insbesondere extrakorporalen Verfahren wie Hämodialyse, Hämofiltration, Hämodiafiltration, Peritonealdialyse, unspezifische Filter wie Cytosorb und spezifische, Ribonuclease enthaltende Einheiten die extrakorporal Zielmoleküle spalten.

21. In-vitro Verfahren zur Diagnose und zum Monitoring der Progredienz einer Nierenerkrankung durch Bestimmung des Ribonucleasegehaltes in Körperflüssigkeiten oder Geweben eines Patienten.


SEQUENCE LISTING

<110>  Pharis Biotec GmbH
       FRIEDRICH-ALEXANDER-UNIVERSITÄT ERLANGEN-NÜRNBERG

<120>  Polypeptid zur Therapie von glomerulären Nierenerkrankungen und
       Analyse des Verlaufes und Prognose der abhängigen Syndrome

<130>  193124EP

<160>  3

<170>  PatentIn version 3.5

<210>  1
<211>  127
<212>  PRT
<213>  human

<400>  1

Lys Glu Ser Arg Ala Lys Lys Phe Gln Arg Gln Met Asp Ser Asp Ser
1               5                   10                  15

Ser Pro Ser Ser Ser Ser Thr Tyr Cys Asn Gln Met Met Arg Arg Arg
            20                  25                  30

Asn Met Thr Gln Gly Arg Cys Lys Pro Val Asn Thr Phe Val His Glu
        35                  40                  45

Pro Leu Val Asp Val Gln Asn Val Cys Phe Gln Glu Lys Val Thr Cys
    50                  55                  60

Lys Asn Gly Gln Gly Asn Cys Tyr Lys Ser Asn Ser Ser Met His Ile
65                  70                  75                  80

Thr Asp Cys Arg Leu Thr Asn Gly Ser Arg Tyr Pro Asn Cys Ala Tyr
                85                  90                  95

Arg Thr Ser Pro Lys Glu Arg His Ile Ile Val Ala Cys Glu Gly Ser
            100                 105                 110

Pro Tyr Val Pro Val His Phe Asp Ala Ser Val Glu Asp Ser Thr
        115                 120                 125

<210>  2
<211>  155
<212>  PRT
<213>  human

<400>  2

Met Ala Leu Glu Lys Ser Leu Val Arg Leu Leu Leu Leu Val Leu Ile
1               5                   10                  15

Leu Leu Val Leu Gly Trp Val Gln Pro Ser Leu Gly Lys Glu Ser Arg
            20                  25                  30

Ala Lys Lys Phe Gln Arg Gln Met Asp Ser Asp Ser Ser Pro Ser Ser
        35                  40                  45

Ser Ser Thr Tyr Cys Asn Gln Met Met Arg Arg Arg Asn Met Thr Gln
    50                  55                  60

Gly Arg Cys Lys Pro Val Asn Thr Phe Val His Glu Pro Leu Val Asp
65                  70                  75                  80

Val Gln Asn Val Cys Phe Gln Glu Lys Val Thr Cys Lys Asn Gly Gln
                85                  90                  95

Gly Asn Cys Tyr Lys Ser Asn Ser Ser Met His Ile Thr Asp Cys Arg
            100                 105                 110

Leu Thr Asn Gly Ser Arg Tyr Pro Asn Cys Ala Tyr Arg Thr Ser Pro
        115                 120                 125

Lys Glu Arg His Ile Ile Val Ala Cys Glu Gly Ser Pro Tyr Val Pro
    130                 135                 140

Val His Phe Asp Ala Ser Val Glu Asp Ser Thr
145                 150                 155

<210>  3
<211>  28
<212>  PRT
<213>  human

<400>  3

Met Ala Leu Glu Lys Ser Leu Val Arg Leu Leu Leu Leu Val Leu Ile
1               5                   10                  15

Leu Leu Val Leu Gly Trp Val Gln Pro Ser Leu Gly
            20                  25